(19) Europäisches Patentamt
European Patent Office
Office européen des brevets

(11) Publication number: 0 174 614
A2

(12) **EUROPEAN PATENT APPLICATION**

(21) Application number: 85111271.4

(22) Date of filing: 06.09.85

(51) Int. Cl.[4]: C 07 C 69/65
C 07 C 51/58, C 07 C 57/52
C 07 C 103/58, C 07 C 121/30
C 08 F 16/24

(30) Priority: 08.09.84 JP 188740/84

(43) Date of publication of application: 19.03.86 Bulletin 86/12

(84) Designated Contracting States: DE FR GB

(71) Applicant: DAIKIN INDUSTRIES, LIMITED
No. 1-12-39, Umeda Kita-ku
Osaka-shi Osaka-fu(JP)

(72) Inventor: Oka, Masahiko
18-6, Hiyoshi-dai 2-chome
Ohtsu-shi Shiga-ken(JP)

(72) Inventor: Yutani, Yuji
1, Kozu 2-chome Minami-ku
Osaka-shi Osaka-ken(JP)

(72) Inventor: Kano, Hideo
21-21, Hitotsuya 2-chome
Settsu-shi Osaka-fu(JP)

(74) Representative: Hoffmann, Klaus, Dr. rer. nat. et al,
Hoffmann . Eitle & Partner Patentanwälte
Arabellastrasse 4
D-8000 München 81(DE)

(54) **Polyfluoroalkenyl ether and polymer comprising the same.**

(57) A polyfluoroalkenyl ether of the formula:

$CFX=CX(CFX)_nO(CFZCF_2O)_mCFYZ$ (I)

wherein X is a chlorine atom or a fluorine atom, Y is a group of the formula:

$-COF$, $-COOM$, $-COOR$, $-CCONR_2$ or $-CN$

in which M is an alkali metal and R is a hydrogen atom or a lower alkyl group, Z is a fluorine atom or a trifluoroalkyl group, n is an integer of 2 to 6 and m is an integer of 0 to 6, which can be homopolymerized or copolymerized with an ethylenically unsaturated comonomer to give a polymer having improved low temperature properties.

EP 0 174 614 A2

Croydon Printing Company Ltd.

# POLYFLUOROALKENYL ETHER AND POLYMER COMPRISING THE SAME

## FIELD OF THE INVENTION

The present invention relates to a polyfluoroalkenyl ether and a polymer comprising the same.

## BACKGROUND OF THE INVENTION

In course of the polymerization of a known ether compound having a vinyl group such as an ether compound of the formula:

$$CF_2=CFOR_f'$$

wherein $R_f'$ is a perfluoroalkyl group, a produced polymer tends to suffer β-cleavage which will terminate the polymerization of the chain by the formation of a CFO group at the polymer molecule end and a new free redical group (cf. U.S. Patent No. 3,642,742 and U.K. Patent No. 1,299,495). To prevent the β-cleavage of the polymer, the polymerization should be carried out at a low temperature. However, at the low polymerization temperature, only a small number of polymerization initiators can be used and the polymerization rate is disadvantageously lowered.

## SUMMARY OF THE INVENTION

One object of the present invention is to provide a novel ether compound having an ethylenically unsaturated bonding and at least two carbon atoms between said unsaturated bonding and etheric oxygen atom, a polymer of which does not suffer β-cleavage during polymerization.

Another object of the present invention is to provide a polymer comprising the novel ether compound.

These and other objects are achieved by a polyfluoroalkenyl ether of the formula:

$$CFX=CX(CFX)_nO(CFZCF_2O)_mCFYZ \quad (I)$$

wherein X is a chlorine atom or a fluorine atom, Y is a group of the formula:

$$-COF,\ -COOM,\ -COOR,\ -CONR_2 \text{ or } -CN$$

in which M is an alkali metal and R is a hydrogen atom or a lower alkyl group, Z is a fluorine atom or a trifluoroalkyl group, n is an integer of 2 to 6 and m is an integer of 0 to 6, and a polymer comprising the same.

## DETAILED DESCRIPTION OF THE INVENTION

In the formula (I), n is usually an integer of 2 to 6, preferably 2 and 3. The ether (I) wherein n is larger than 3 is somewhat expensive and not economical. m is usually an integer of 0 to 6. When m is larger than 6, isolation of each ether compound is difficult in the synthesis steps. In addition, the ether (I) having larger m and thus a larger molecular weight becomes more viscous. The lower alkyl group includes one having 1 to 4 carbon atoms, preferably 1 to 3 carbon atoms.

Among the various ethers according to the present invention, the ether (I) wherein X is a fluorine atom, n is 2 or 3 and m is an integer of 0 to 3 is preferable.

Specific examples of the ether (1) are as follows:

$CF_2=CFCF_2CF_2OCF(CF_3)COF$

$CF_2=CFCF_2CF_2OCF(CF_3)COOCH_3$

$CF_2=CFCF_2CF_2OCF(CF_3)COOH$

$CF_2=CFCF_2CF_2OCF(CF_3)CONH_2$

$CF_2=CFCF_2CF_2OCF(CF_3)CON(CH_3)_2$

$CF_2=CFCF_2CF_2OCF(CF_3)CN$

$CF_2=CFCF_2CF_2O[CF(CF_3)CF_2O]_2CF(CF_3)COF$

$CF_2=CFCF_2CF_2O[CF(CF_3)CF_2O]_2CF(CF_3)COOCH_3$

$CFCl=CFCF_2CF_2OCF(CF_3)COF$

$CFCl=CFCF_2CF_2OCF(CF_3)COOCH_3$.

The ether (I) wherein Y is a group of the formula: -COOR in which R is a hydrogen atom or a lower alkyl group and Z is a trifluoroalkyl group may be prepared according to the following reaction scheme:

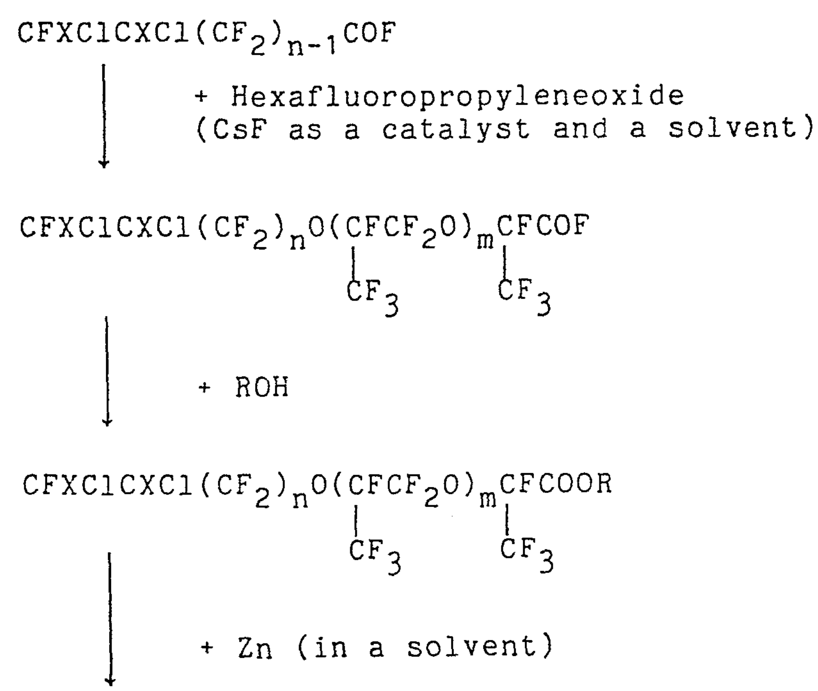


(II)

(III)

(IV)

$$CFX=CX(CF_2)_nO(\underset{CF_3}{\underset{|}{CF}}CF_2O)_m\underset{CF_3}{\underset{|}{CF}}COOR \qquad (V)$$

The ether (I) wherein Y is a group of the formula: -COOM, -CN or $-CONR_2$ in which M and R are the same as defined above may be prepared by subjecting the compound (III) to a suitable subsequent treatment. For example, the ether (I) wherein Y is $-CONR_2$ is prepared by reacting the compound (III) with $NHR_2$. The ether (I) wherein Y is -COOM is prepared by reacting the compound (III) with an alkali metal hydroxide. The ether (I) wherein Y is -CN is prepared by reacting the compound (III) with ammonia followed by dehydration.

Specific examples of the solvent used in the conversion of the compound (II) to the compound (III) are glymes (e.g., ethyleneglycol dimethyl ether, diethyleneglycol dimethyl ether and tetraethyleneglycol dimethyl ether) and acetonitrile. Specific examples of the solvent used in the conversion of the compound (IV) to the compound (V) are alcohols (e.g., methanol, ethanol and ethyleneglycol) and amides (e.g., dimethylformamide and dimethylacetamide).

The catalyst used in the conversion of the compound (II) to the compound (III) includes cesium fluoride, potassium fluoride, silver fluoride, ammonium fluoride, tetra($C_1$-$C_4$ alkyl)ammonium fluoride (e.g, tetramethylammonium fluoride, tetraethylammonium fluoride and tetrabutylammonium fluoride), sulfonium fluoride (e,g., benzenesulfonium fluoride), etc. A preferred example of the catalyst used in the conversion of the compound (IV) to the compound (V) is metal zinc.

In each reaction step, the reaction temperature and the reaction time vary with the kind of the desired product. Preferred reaction conditions are as follows:

The conversion of the compound (II) to the compound (II) is carried out at a temperature from -30°C to +30°C for 3 to 20 hours.

The compound (III) is reacted with the alcohol by dropwise adding the latter to the former while cooling the reaction system with iced water to obtain the compound (IV).

The conversion of the compound (IV) to the compound (V) is carried out at a refluxing temperature of the solvent for 1 to 5 hours.

Although the polyfluoroalkenyl ether (I) of the invention can be homopolymerized, it is preferably copolymerized with at least one ethylenically unsaturated monomer so as to modify characteristics (e.g., low temperature properties) of a homopolymer of the ethylenically unsaturated monomer.

The polymer of the invention includes a homopolymer of the ether (I), a copolymer of at least two different kinds of the ethers (I) and a copolymer of the ether (I) with the ethylenically unsaturated comonomer.

The ethylenically unsaturated comonomer may be any one that can be copolymerized with the ether (I) and includes a fluorine-not-containing ethylenically unsaturated compound (e.g., ethylene, vinyl chloride, vinylidene chloride, acrylic acid and methacrylic acid), and a fluorine-containing ethylenically unsaturated compound (e.g., tetrafluoroethylene (hereinafter referred to as "TFE"), trifluoroethylene, vinylidene fluoride (hereinafter referred to as "VdF"), vinyl fluoride, hexafluoropropylene (hereinafter referred to as "HFP"), chlorotrifluoroethylene, perfluoro(methyl vinyl ether) and perfluoro(propyl vinyl ether)). In view of making the best use of the characteristics of the polyfluoroalkenyl ether (I) of the invention, it is preferably copolymerized with the fluorine-containing ethylenically unsaturated compound.

The copolymer of the polyfluoroalkenyl ether (I) with the comonomer preferably contains 0.1 to 50 % by mole of the former from view points of properties of the copolymer.

The polymerization is carried out under substantially the same conditions as in polymerization of the conventional fluoroolefins. It may be carried out in a suitable manner, for example, bulk, suspension or emulsion polymerization in the presence of a polymerization initiator.

The polymerization is preferably carried out in the presence of a solvent to improve the contact between the monomer molecules. Preferable examples of the solvent are halogenated hydrocarbons (e.g., trichlorotrifluoroethane, dichlorotetrafluoroethane, trichlorofluoromethane, dichlorodifluoromethane and perfluorocyclobutane) and water.

As the polymerization initiator, any one that does not deteriorate the properties of the produced polymer may be used. Specific examples of the polymerization initiator are di(fluoroacyl)peroxides and di(chlorofluoroacyl)-peroxides as described in Japanese Patent Publication Nos. 28675/1974 and 44301/1972, dialkylperoxydicarbonates (e.g., diisopropylperoxydicarbonate), diacylperoxides (e.g., isobutyrylperoxide), and peroxyesters (e.g., t-butyl oxyisobutyrate and t-butyl peroxypivalate).

Optionally, a chain transfer agent which is used in conventional radical polymerization may be added to the polymerization system according to the present invention. Specific examples of the chain transfer agent are isoparaffin, tetrachloromethane, dimethyl malonate, diethyl ether, mercaptans, alcohols and so forth.

The reaction temperature is not critical in the polymerization according to the present invention. Preferred range of the reaction temperature is between a room temperature and 100°C. The reaction pressure may be autogenic pressure of the used monomer(s), although the polymerization system may be pressurized.

The copolymer of the invention, particularly a copolymer of the ether (I) and VdF, is an elastomer having good low temperature properties. For example, the copolymer with VdF has about 10°C lower freezing temperature than the conventional VdF/HFP copolymer and the VdF/TFE/HEP terpolymer which have glass transition temperatures from -20°C to -15°C. Thus, the copolymer of the invention is useful as a sealing material to be used at a low temperature. In addition, the copolymer of the invention does not foam during molding at a high temperature, which results in easy and stable molding.

The copolymer comprising the ether (I) wherein Y is -COOM is useful as an ion exchanger. When the copolymer of the invention comprises the ether (I) wherein Y is -CN, the ciano groups act as cross linking sites. The cross linking may be effected by heating the elastomer in the presence of a conventional catalyst such as tetraphenyltin.

## PREFERRED EMBODIMENTS OF THE INVENTION

The present invention will be hereinafter explained further in detail by following Examples.

### Example 1

Preparation of

$$CF_2{=}CF(CF_2)_2O(\underset{CF_3}{CF}CF_2O)_2\underset{CF_3}{CF}COOCH_3$$

(A) To a 2 liter flask containing cesium fluoride (15 g) and absolute tetraglyme (100 ml), 2,3-dichloropentafluorobutanoyl fluoride (180 g) was added. While keeping a temperature of the flask interior at a temperature from -15°C to -20°C with stirring, hexafluoropropyleneoxide (hereinafter referred to as "HFPO") was injected at such a rate that the mixture was refluxed by a condenser cooled with dry ice. After 4 hours from the initiation of the reaction, the injection of HFPO was terminated and the reaction mixture was stirred for additional 30 minutes. Then, a layer of the product was separated off by means of a separating funnel. To the product, methanol (48 g) was added with cooling by iced water to obtain a compound of the formula:

$$CF_2ClCFCl(CF_2)_2O(\underset{CF_3}{\underset{|}{CF}}CF_2O)_m\underset{CF_3}{\underset{|}{CF}}COOCH_3$$

According to gaschromatographic analysis of the product, the proportion of the compounds having different m were as follows:

| m | Mole % |
|---|---|
| 0 | 1.56 |
| 1 | 5.07 |
| 2 | 41.12 |
| 3 | 46.02 |
| 4 | 6.22 |

(B) Rectification of the product in the step (A) afforded a slightly viscous colorless transparent liquid (178.5 g) of the formula:

$$CF_2ClCFCl(CF_2)_2O(\underset{|\atop CF_3}{CF}CF_2O)_2\underset{|\atop CF_3}{CF}COOCH_3 \quad .$$

Boiling point 155°C/92 mmHg.

IR absorption spectrum showed strong absorption at 1,785 $cm^{-1}$ assigned to C=O bonding in the ester group and absorption at 2,980 $cm^{-1}$ assigned to C-H bonding in the methyl groups.

Elemental analysis ($C_{14}Cl_2F_{23}H_3O_5$):

| | C | Cl | F | H |
|---|---|---|---|---|
| Calc'd: | 22.13 | 9.35 | 57.58 | 0.40 |
| Found: | 21.86 | 9.01 | 58.22 | 0.32 |

(C) To a four-necked 500 ml flask directly connected to a rectifier, powdery metal zinc (35 g) and dimethylformamide (200 ml) were charged, and the compound produced in the step (B) (156.9 g) was dropwise added in a boiling state of dimethylformamide while collecting distillate. After the addition of the compound was finished, the stirring was continued to complete the reaction. The distillate was washed with water and rectified to obtain the titled compound (94.3 g). Boiling point 103°C/28 mmHg. Yield 66 %.

| Elemental analysis ($C_{14}F_{23}H_3O_5$): | C | F | H |
|---|---|---|---|
| Calc'd: | 22.42 | 63.52 | 0.44 |
| Found: | 24.00 | 64.11 | 0.39 |

Example 2

Preparation of a copolymer of the compound prepared in Example 1 and VdF

In a 100 ml glass pressure reactor equipped with a valve, the compound prepared in Example 1 (hereinafter referred to as "$\phi_2$BE") (10.3 g), 1,1,2-trichloro-1,2,2-trifluoroethane (hereinafter referred to as "R-113") (10.0 ml) and a solution of 2,4,5-trichloroperfluorohexanoylperoxide in R-113 (0.44 g/ml) (0.8 g) were charged and cooled by dry ice/methanol, and the reactor interior was replaced with VdF. Then, VdF (5.2 g) was injected and reacted at 20±1°C for 12 hours with shaking. As the reaction proceeded, the interior pressure dropped from 8.1 $kg/cm^2G$ to 2.2 $kg/cm^2G$ and the reaction mixture was clouded. After discharging the unreacted monomers, the reaction mixture was recovered, washed with diethyl ether three times and dried under reduced pressure to obtain the copolymer (8.4 g). Molar ratio of VdF : $\phi_2$BE = 90.0:10.0 (according to $^{19}$F-NMR). [η] (35°C, in MEK) = 0.32. Glass transition temperature -25°C.

Example 3

In the same manner as in Example 2 but charging 2.6 g of VdF and proceeding the reaction for 9 hours, the

reaction was carried out, during which the interior pressure dropped from 4.2 kg/$cm^2$G to 1.8 kg/$cm^2$G, to obtain the copolymer (3.4 g). Molar ratio of VdF : $\phi_2$BE = 84:16. Glass transition temperature -27.3°C.

Example 4

In the same manner as in Example 2 but replacing the reactor interior with TFE, charging TFE (1.4 g) in place of VdF and proceeding the reaction for 28 minutes, the reaction was carried out, during which the interior pressure dropped from 1.8 kg/$cm^2$G to 1.2 kg/$cm^2$G, to obtain the copolymer (1.9 g). Melting point 326.3°C.

Example 5

In the same manner as in Example 2 but replacing the reactor interior with ethylene, charging ethylene (0.8 g) in place of VdF and proceeding the reaction for 19 hours, the reaction was carried out, during which the interior pressure dropped from 3.8 kg/$cm^2$G to 3.4 kg/$cm^2$G, to obtain the copolymer (1.2 g). Molar ration of ethylene : $\phi_2$BE = 84.2:15.8.

CLAIMS

1. A polyfluoroalkenyl ether of the formula:

$$CFX{=}CX(CFX)_nO(CFZCF_2O)_mCFYZ \qquad (I)$$

wherein X is a chlorine atom or a fluorine atom, Y is a group of the formula:

-COF, -COOM, -COOR, $-CONR_2$ or -CN

in which M is an alkali metal and R is a hydrogen atom or a lower alkyl group, Z is a fluorine atom or a trifluoroalkyl group, n is an integer of 2 to 6 and m is an integer of 0 to 6.

2. A polyfluoroalkenyl ether according to claim 1, wherein X is a fluorine atom, n is 2 or 3 and m is an integer of 0 to 3.

3. A polyfluoroalkenyl ether according to claim 1, wherein Y is a group of the formula: -COF.

4. A polyfluoroalkenyl ether according to claim 1, wherein Y is a group of the formula: -COOM in which M is an alkali metal.

5. A polyfluoroalkenyl ether according to claim 1, wherein Y is a group of the formula: -COOR in which R is a lower alkyl group.

6. A polyfluoroalkenyl ether according to claim 1, wherein Y is a group of the formula: $-CONR_2$ in which R is a lower alkyl group.

7. A polyfluoroalkenyl ether according to claim 1, wherein Y is a cyano group.

8. A polymer comprising repeating units derived from a polyfluoroalkenyl ether (I) according to claim 1.

9. A polymer according to claim 9, which is a homopolymer of the polyfluoroalkenyl ether (I).

10. A polymer according to claim 9, which is a copolymer of at least two different kinds of the polyfluoroalkenyl ether (I).

11. A polymer according to claim 9, which is a copolymer of the polyfluoroalkenyl ether (I) with at least one ethylenically unsaturated comonomer.

12. A polymer according to claim 11, wherein the comonomer is a fluorine-not-containing ethylenically unsaturated compound.

13. A polymer according to claim 11, wherein the comonomer is a fluorine-containing ethylenically unsaturated compound.